# EUROPEAN PATENT APPLICATION

(11) **EP 1 155 682 A1**
(43) Date of publication of application: **21.11.2001**
(21) Application number: 01203157.1
(22) Date of filing: 18.08.1995
(51) Int. Cl.: A61K 7/20

(54) **Oral composition with an improved teeth whitening effect**

(30) Priority: 22.08.1994 EP 94306191
(62) Divisional of application: 95930506.1
(71) Applicant: UNILEVER N.V., 3013 AL Rotterdam (NL); UNILEVER PLC, London EC4P 4BQ (GB)
(72) Inventor: Thornthwaite, David William, Bebington, Wirral Merseyside CH63 3JW (GB); Waterfield, Philip Christopher, Bebington, Wirral Merseyside CH63 3JW (GB); De Ponti, Rita Cristina, 20151 Milan (IT)
(74) Representative: Newbould, Frazer Anthony

(57) **Abstract**

The present invention relates to oral care compositions with an improved teeth whitening effect. This effect is achieved by inclusion in the oral care compositions of certain organic peroxy acids as teeth whitening/bleaching agents.

## Description

The present invention relates to an oral composition with an improved teeth whitening effect. More particularly, it relates to an oral composition with an improved teeth whitening effect comprising a safe and effective amount of certain organic peroxyacids.

The use of peroxy compounds in oral care compostions has already been proposed in the prior art. Many peroxy compounds have been suggested for whitening/bleaching human teeth, and representative examples of such peroxy compounds are hydrogen peroxide, urea peroxide, organic peracids such as perphthalic acid, diperoxycarboxylic acids, 1,12-dodecanedioic peroxy acid, peroxy acetic acid and systems comprising a peroxy compound and a peroxy acid precursor which generate peroxy acetic acid in situ, such as sodium perborate and tetraacetylethylene diamine (TAED). The use of peroxy acetic acid is suggested in particular in e.g. EP-A-0545,594 (Colgate), which also sets out the various prior proposals, made in the art for several peroxy compounds as bleaching/whitening agent for human teeth.

We have now found that certain organic peroxy acids, which will be defined hereinafter, are either more stable than peroxy acetic acid or are more effective than peroxy acetic acid. These certain organic peroxy acids are selected from the group consisting of:
1) peroxy amido phthalamides having the structural formula: 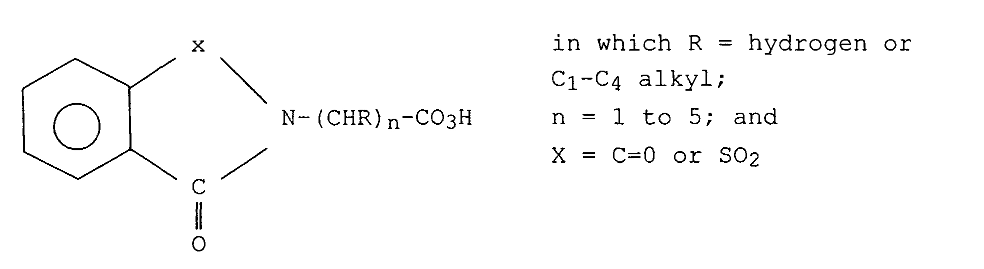
2) cationic peroxy acids having the structural formula: 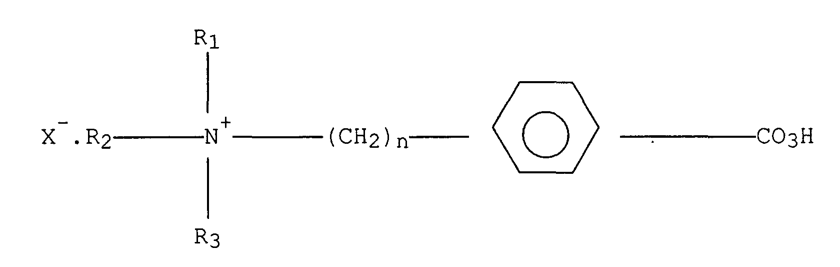 wherein: R₁ is a, optionally substituted, C₁-C₇ alkyl or alkenyl or alkaryl with a C₁-C₇ alkyl group; R₂ and R₃, are each independently a C₁-C₃ alkyl or C₁-C₃ substituted alkyl group; n is an integer from 0 to 3; and X⁻ is a suitable counter-anion such as methylsulphonate or a surfactant anion selected from alkyl carboxylates; alkyl ether sulphates; alkylbenzene sulphonates; C₁₂₋₁₅ primary and C₁₄₋₁₈ secondary alkyl sulphates; olefin sulphonates; alkane sulphonates, dialkyl sulphosuccinates; fatty acid ester sulphonates; alkylether sulphonates, alkylether carboxylates; sulphonated alkyl polyglycosides; sulphonated alkanoyl glucose ethers, sulphonated monoglycerol ethers; secondary alkane sulphonates; esterified isethionates; alkyl and dialkyl phosphates; sodium fatty acid sulphonates; and fatty acid soaps.
3) cationic peroxycarboxylic acids having the formula 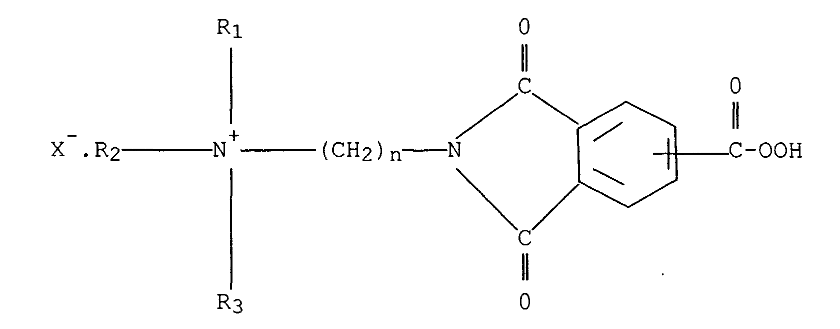 wherein R₁, R₂ and R₃ are each independently a C₁-C₇ alkyl group or C₁-C₇ substituted alkyl group, n is an integer of from 2 to 10 and X is a counter anion.
4) cationic peroxyacids of formula 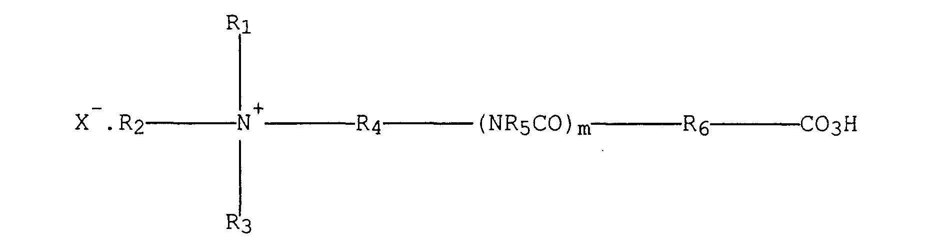 wherein:
   R₁ is a, optionally substituted, C₁-C₂₄ alkyl or alkenyl or alkylaryl with a C₁-C₂₄ alkyl group;
   R₂ and R₃ are each independently a C₁-C₃ alkyl or C₁-C₃ substituted alkyl group;
   R₄ and R₆ are each independently aryl or (CH₂)ₙ where n is an integer from 1 to 7;
   R₅ is selected from hydrogen, C₁-C₇ alkyl, or aryl substituted with a C₁-C₂ alkyl;
   m = 0 or 1, whereby when m = 0, R₄ = C₁-C₃ alkylgroup; and
   X is a counter anion.
5) butyl imido peroxytrimellitic acid ("BIPTA")
6) 6, 6'-terephthal-di(amidoperoxyhexanoic)acid ("TPCAP")
7) monononylamide of peroxyadipic acid ("NAPAA") and mixtures thereof.

The peroxy amido phthalamides of formula 1) are known per se and have been described in EP-A-325,288 and EP-A-325,289. A preferred compound of this formula is N-phthalimido hexanoic peroxy acid ("PAP") of formula 1), in which R = H, n = 5 and X = C=0. An example of a compound according to formula 1)
wherein x = SO₂ is saccharin-perhexanoic acid ("saccharin PAP"), as described in EP-A-485,927.

The cationic peroxyacids of formula 2) are also known per se, and have been described in WO-A-94/10399. A preferred compound is a quaternary benzylperoxyacid ("QBPA"), of the general formula 2) in which R₁, R₂ and R₃ are each methyl, n = 1 and X = methylsulphonate.

The cationic peroxyacids of formula 3) are equally known per se, and have been described in EP-A-508,623. A preferred compound is trimethyl ammonium propenyl imidoperoxy-mellitic acid of formula 3), in which R₁, R₂ and R₃ are each methyl, n = 3 and X = methanesulphonate.

The cationic peroxyacids of formula 4) are novel compounds; they are the subject of our co-pending application GB 9305863.4, filed 22 March 1993. A preferred compound of this formula 4) is 3-trimethyl-ammonium-propyl-amido-4'-peroxybenzoic acid, of formula 4) in which R₁, R₂ and R₃ are methyl, R₄ is propyl, R₅ is hydrogen, R₆ is benzyl and X is methosulphate.

BIPTA is described in WO/91/09843; TPCAP is described in US-A-5,220,052, and NAPAA is described in EP-A-349,220.

Thus, the present invention relates to an oral composition with an improved teeth whitening effect, comprising a safe and effective amount of an organic peroxy acid as bleaching/whitening agent, characterised in that the organic peroxy acid is selected from the group consisting of peroxyacids 1) - 7) as defined above.

Preferably, the peroxyacid is PAP and/or QBPA. Compared with peroxy acetic acid, PAP is, on an equivalent molar ratio, as effective but more stable, whereas QBPA is more effective than peroxy acetic acid.

The amount of peroxyacids,used in the present invention, may vary from 0.01 to 99 % by weight of the oral composition, preferably from 0.1 to 30 % by weight, particularly preferably from 0.1-5 % by weight.

The oral compositions can be formulated in any suitable application form, such as gels, mouthwashes, toothpowders and toothpastes. They may be formulated into a single formulation or they may be formulated for multi compartment containers into different formulations, e.g. one containing the peroxy bleach and ingredients compatible therewith, and another containing the remaining ingredients.

The oral care compositions of the present invention may furthermore comprise optional, conventional ingredients such as pharmaceutically acceptable carriers like starch, sucrose, water or water/alcohol systems etc.. Small amounts of surfactants may also be included, such as anionic, nonionic and amphoteric surfactants. When formulated into a dentifrice, such formulation may contain all the usual dentifrice ingredients.

Thus, they may comprise particulate abrasive materials such as silicas, aluminas, calcium carbonates, dicalciumphosphates, calcium pyrophosphates hydroxyapatites, trimetaphosphates, insoluble hexametaphosphates and so on, usually in amounts between 5 and 60 % by weight.

Furthermore, the dentifrice formulations may comprise humectants such as glycerol, sorbitol, propyleneglycol, xylitol, lactitol and so on.

Binders and thickeners such as sodium carboxymethylcellulose, xanthan gum, gum arabic etc. may also be included, as well as synthetic polymers such as polyacrylates and carboxyvinyl polymers such as Carbopol® .

Flavours such as peppermint and spearmint oils may also be included, as well as preservatives, opacifying agents, colouring agents, pH-adjusting agents, sweetening agents and so on. Stabilising agents for the organic peroxy acids such as dipicolinic acid or sodium stannate may also be usefully included.

Anti-bacterial agents may also be included such as Triclosan, chlorhexidine, copper-, zinc- and stannous salts such as zinc citrate, sodium zinc citrate and stannous pyrophosphate, sanguinarine extract, metronidazole. Further examples of anti-bacterial agents are quaternary ammonium compounds such as cetylpyridinium chloride; bis-guanides such as chlorhexidine digluconate, hexetidine, octenidine, alexidine; halogenated bisphenolic compounds such as 2,2' methylenebis-(4-chloro-6-bromophenol).

Polymeric compounds which can enhance the delivery of active ingredients such as anti-bacterial agents can also be included. Examples of such polymers are copolymers of polyvinylmethylether with maleic anhydride and other similar delivery enhancing polymers, e.g. those described in DE-A-3,942,643 (Colgate).

Furthermore anti-inflammatory agents such as ibuprofen, flurbiprofen, aspirin, indomethacin etc. may also be included.

Anti-caries agents such as sodium- and stannous fluoride, aminefluorides, monosodiumfluorophosphate, casein, plaque buffers such as urea, calcium lactate, calcium glycerophosphate, strontium polyacrylates may also be included. Other optional ingredients include vitamins such as Vitamin C, and plant extracts. Desensitising agents such as potassium citrate, potassium chloride, potasium tartrate, potassium bicarbonate, potassium oxalate, potassium nitrate as well as strontium salts may also be included.

Buffers and salts to buffer the pH and ionic strength of the compositions may also be included. Liposomes and other encapsulates may also be used to improve delivery or stability of active ingredients.

Furthermore, the oral compositions may comprise anticalculus agents such as alkalimetal pyrophosphates, hypophosphite-containing polymers, organic phosphonates, phosphocitrates etc..

In addition, the compositions may comprise functional biomolecules such as bacteriocins, antibodies, enzymes and so on.

Other optional ingredients that may be included are e.g. bleaching agents, e.g. those described in EP-A-0 545,594, effervescing systems such as sodium bicarbonate/citric acid systems, colour change systems, and so on.

When formulated as a mouthwash, the oral care composition usually comprises a water/alcohol solution, flavour, humectant, sweetener and colorant.

Since the peroxyacids of the invention also have an antimicrobial property, the composition of the invention are also effective to combat plaque and caries.

The present invention will further be illustrated by way of Example.

### Example I

PAP and QBPA were evaluated as to their bleaching efficacy.

The bleaching agents were evaluated as follows:
(1) Synthetic hydroxyapatite discs were polished and placed in sterile saliva at 37°C overnight to form a pellicle.
(2) Discs were stained with tea/coffee/iron salts/saliva mixture for seven days at 37°C.
(3) Stained discs were immersed in bleaching solutions for desired time.
(4) The change in colour of the discs was measured using a Minolta chromameter CR-300 in L*a* b* mode. Using L^{*} (initial), L* (soiled), and L* (cleaned), the percentage of stain removed was calculated.

A comparison was made with 0.1% peroxy acetic acid, and 0.5M NaHCO₃ was used as control. The concentration of PAP was 0.37%, and for QBPA (with bicarbonate) 0.4% (compared with 0.1% peroxy acetic acid/0.1M NaOH).

| Time (mins) | % stain removed with | | |
|---|---|---|---|
| | PAP | Peroxyacetic acid | NaHCO₃ |
| 0 | 0 | 0 | 0 |
| 1 | 52 | 46 | -7 |
| 3 | 76 | 68 | -5 |
| 5 | 84 | 79 | 7 |

| Time (mins) | % stain removed with | | |
|---|---|---|---|
| | QBPA | Peroxyacetic acid | NaHCO₃ |
| 0 | 0 | 0 | 0 |
| 1 | 71 | 25 | -21 |
| 3 | 92 | 54 | -23 |
| 5 | 96 | 68 | -23 |

### Example II

Various levels of PAP were tested according to the method of Example I. The following results were obtained:

### pH = 7

| Time (mins) | % stain removed with | | | | |
|---|---|---|---|---|---|
| | 2 % | 1.5 % | 1.0 % | 0.5 % | 0 % PAP |
| 0 | 0 | 0 | 0 | 0 | 0 |
| 1 | 30 | 29 | 24 | 13 | 1 |
| 3 | 48 | 42 | 48 | 28 | 2 |
| 5 | 59 | 53 | 60 | 38 | 5 |

### pH = 4.5

| Time (mins) | % stain removed with | | | | |
|---|---|---|---|---|---|
| | 2 % | 1.5 % | 1.0 % | 0.5 % | 0 % PAP |
| 0 | 0 | 0 | 0 | 0 | 0 |
| 1 | 15 | 23 | 15 | 14 | 1 |
| 3 | 29 | 39 | 30 | 27 | 2 |
| 5 | 38 | 50 | 38 | 39 | 5 |

### Example III

The method of Example I was used to test the effect of 0.82 % PAP at different pH-values. The following results were obtained:

| Time (mins) | % stain removed with | | | |
|---|---|---|---|---|
| | pH 4.5 | pH 6.0 | pH 7.0 | pH 8.0 |
| 0 | 0 | 0 | 0 | 0 |
| 1 | 13 | 17 | 16 | 22 |
| 3 | 22 | 33 | 35 | 41 |
| 5 | 32 | 42 | 46 | 50 |

### Example IV

The method of Example I was used to test the effect of the formulations given below:

The following products were prepared:

| **Phase A** | | **Phase B** | **%** |
|---|---|---|---|
| PAP | 2 % | Sodium Bicarbonate | 10 |
| Silicas | | Glycerine | 35 |
| (Cab-0-sils) | 7 % | Silicas | 18 |
| Dipicolinic acid | 0.27 | Monosodium Phosphate | 1 |
| Na₂H₂P₂O₇ | 0.55 | Polyethyleneglycol (MW1500) | 5 |
| H₃PO₄ | 0.25 | | |
| H₂O | 90.00 | H₂O | 31 |

Test formulation 1) was made of a slurry of 25 % A + 25 % B + 50 % H₂O.

Test formulation 2) was made of a slurry of 50 % B + 50 % H₂O.

The following results were obtained:

| Time (mins) | % stain removed with | |
|---|---|---|
| | 1) | 2) |
| 0 | 0 | 0 |
| 2 | 51 | 6 |
| 5 | 64 | 29 |
| 10 | 77 | 35 |

### Example V

PAP and NaHCO₃ were compared in an in situ stain removal test for 3 mins.

The test protocol was as follows:
* Pieces of Bovine enamel were attached to partial or full dentures, and
* Worn in the mouth to allow a 21 day stain to build up.

The dentures were then soaked in:
* Solutions of agents for 3 mins.
* The change in stain intensity was then measured with a Minolta chromameter CR-321 in L*a*b* mode, and the
* % stain removed calculated.

The following results were obtained:

| | **% stain removal** |
|---|---|
| with NaHCO₃ | 0 |
| with 1 % PAP/NaHCO₃ | 32. |

For comparison purposes, combinations of H₂O₂ and NaHCO₃ gave the following results:

| | **% stain removal** |
|---|---|
| with 1 % H₂O₂/NaHCO₃ | 20 |
| with 3 % H₂O₂/NaHCO₃ | 24 |

### Example VI

1. Various peracids were tested according to the method of Example I.
The concentrations of peracids were all 36mM made up in 0.5M sodium bicarbonate.

| Time (mins) | | % stain removed with | | |
|---|---|---|---|---|
| | BIPTA | DPDDA | NAPAA | PAP |
| 0 | 0 | 0 | 0 | 0 |
| 1 | 25 | 16 | 16 | 13 |
| 3 | 39 | 32 | 29 | 28 |
| 5 | 51 | -- | 40 | 39 |

BIPTA = butyl imido peroxytrimellitic acid
DPDDA = 1,10 Diperoxydodecandioic acid (for comparison; DPDDA is not according to the invention)
NAPAA = monononylamide of peroxyadipic acid
PAP = N-phthalimido hexanoic acid.
2. The following peracids were tested according to the method of Example I. The concentrations of the peracids were all 36mM made up in 0.5M sodium bicarbonate.

| Time (mins) | | % stain removed with | | |
|---|---|---|---|---|
| | MPPA | TPCAP | PAP | NaHCO₃ |
| 0 | 0 | 0 | 0 | 0 |
| 1 | 10 | 18 | 20 | -8 |
| 3 | 25 | 39 | 52 | -9 |
| 5 | 38 | 53 | 67 | -5 |

MPPA = monoperoxyphthalic acid (for comparison: MPPA is not according to the invention)
TPCAP = 6,6'-terephthal-di(amidoperoxyhexanoic)acid.
3. PAP was compared with a bleach precursor system according to EP 545,594.
The concentrations were as follows; PAP 30mM, H₂O₂ 30mM, GPA (= glucosepentaacetate) 128mM in 0.5M bicarbonate.

| Time (mins) | % stain removed with | | |
|---|---|---|---|
| | PAP | H₂O₂/GPA | NaHCO₃ |
| 0 | 0 | 0 | 0 |
| 1 | 23 | 21 | 0 |
| 3 | 48 | 43 | 1 |
| 5 | 63 | 54 | 7. |

### Example VII

The storage stability of various formulations with 2 % PAP, further containing thickening silica, a binding agent, water and a humectant, which formulations contained 0.1 % dipicolinic acid as stabiliser or 0.01 % sodium stannate as stabiliser, was tested at various temperatures and various storage times.

Overall, the formulations without a stabiliser were stable at 5 °C and at room temperature over a period of 2 months. The inclusion of the above stabilising agents did give a slight improvement after 6 months. At 37 °C, both stabilisers did give an improvement in the storage stability of the PAP after 4 months.

### Example VIII

The method of Example I was used to test the effect of the formulations given below:

The following products were prepared:

### Product A

| Ingredient | % W/W |
|---|---|
| Glycerol | 35 |
| Colouring agent | 0.5 |
| Polyethyleneglycol (MW1500) | 5 |
| Sodium lauryl sulphate | 2.4 |
| Formalin | 0.1 |
| Thickening silica | 12 |
| Sodium carboxymethylcellulose | 0.8 |
| NaHCO₃ | 10 |
| H₂O | To 100 |

### Product B

| Ingredient | % W/W |
|---|---|
| H₃PO₄ (85 %) | 0.663 |
| PAP | 6 |
| NaH₂PO₄.2H₂O | 0.1 |
| NaOH(2N) | 2 |
| Thickening silica | 8 |
| H₂O | To 100 |

### Product C

| Ingredient | % W/W |
|---|---|
| H₃PO₄ (85 %) | 0.663 |
| NaH₂PO₄.2H₂O | 0.1 |
| Formalin | 0.1 |
| Thickening silica | 12 |
| H₂O | To 100 |

Test formulation 1) was made of 50 % B and 50 % A.
Test formulation 2) was made of 50 % C and 50 % A.

The results obtained are shown below:

| | **% Stain Removal with** | |
|---|---|---|
| **Time (mins)** | **1)** | **2)** |
| O | 0 | 0 |
| 2 | 56 | 20 |
| 5 | 69 | 26 |
| 10 | 73 | 31 |

## Claims

1. An oral composition with an improved teeth whitening effect, comprising a safe and effective amount of an organic peroxy acid, **characterised in that** the organic peroxy acid is
a cationic peroxyacid of formula 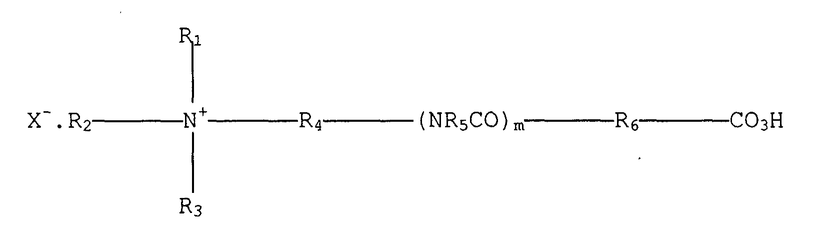 wherein:
R₁ is a, optionally substituted, C₁-C₂₄ alkyl or alkenyl or alkylaryl with a C₁-C₂₄ alkyl group;
R₂ and R₃ are each independently a C₁-C₃ alkyl or C₁-C₃ substituted alkyl group;
R₄ and R₆ are each independently aryl or (CH₂)ₙ where n is an integer from 1 to 7;
R₅ is selected from hydrogen, C₁-C₇ alkyl, or aryl substituted with a C₁-C₂ alkyl;
m = 0 or 1, whereby when m = 0, R₄ = C₁-C₃ alkylgroup; and
X is a counter anion.

2. Use of the peroxyacids of claim 1 as teeth whitening agent in the manufacture of an oral composition having improved teeth whitening properties.
